## Europäisches Patentamt
## European Patent Office
## Office européen des brevets

(11) Publication number: **0 101 418**
**B1**

(12) **EUROPEAN PATENT SPECIFICATION**

(45) Date of publication of patent specification: 27.12.90

(21) Application number: 83850147.6

(22) Date of filing: 31.05.83

(51) Int. Cl.⁵: **A 61 K 9/10,** A 61 K 9/50,
A 61 K 31/52, A 61 K 31/43

(54) Pharmaceutical mixture.

(30) Priority: 24.06.82 SE 8203953

(43) Date of publication of application:
22.02.84 Bulletin 84/08

(45) Publication of the grant of the patent:
27.12.90 Bulletin 90/52

(84) Designated Contracting States:
AT BE CH DE FR IT LI LU NL SE

(56) References cited:
EP-A-0 069 097
GB-A-2 082 539
US-A-3 109 775

(73) Proprietor: Astra Läkemedel Aktiebolag
Strängnäsvägen 44
S-151 85 Södertälje (SE)

(72) Inventor: Källstrand, Anders Göran Vilhelm
Vängavägen 16
S-240 10 Dalby (SE)
Inventor: Mattsson, Kjell Johan
S:ta Ragnhildsgatan 42b
S-151 34 Södertälje (SE)
Inventor: Sjöqvist, Rolf Ivar
Polonäsvägen 1
S-154 00 Gnesta (SE)

(74) Representative: Danielsson, Sten Ove et al
AB ASTRA Patent and Trademark Department
S-151 85 Södertälje (SE)

The file contains technical information
submitted after the application was filed and
not included in this specification

Courier Press, Leamington Spa, England.

## Description

The present invention is concerned with an oral pharmaceutical preparation containing an microencapsulated pharmaceutically active substance. More specifically the preparation is a dry powder for mixture or said dry powder dissolved in an aqueous solution.

The object of the invention is to provide a preparation wherein the dissolution of the active substance from the microencapsulation is controlled, which is a ready-to-use pharmaceutical suspension with controlled release of active substance including masking of bad taste and stability increasing of active substance characterized in that it contains a microencapsulated active substance combined outside the encapsulation with 60—99% (weight/weight) of the ready-to-use suspension, preferably 60—75%, of a release controlling substance which is a carbohydrate selected from polysaccharides, oligosaccharides, disaccharides and monosaccharides or a carbohydrate-related compound selected from the group consisting of mannitol, sorbitol, glycerol, glycol, a glycoside of a monosaccharide and a substance derived from ethyleneglycol or a mixture of such compounds.

Background art

Among alternative forms of orally administering pharmaceutically active substances the use of a solution or a suspension of the active principle in an aqueous solution is a form often seen in pediatric use. This preparation is called a mixture. The dry powder including the active principle and adjuvants which is to be dissolved or suspended is called dry powder for mixture.

The preparation is stored as a dry powder. Before administration the dry powder is dissolved or suspended in an aqueous solution giving rise to a liquid formulation for oral administration—a mixture. Alternatively the mixture can be prepared in the factor and stored at least for two years prior to administration. Pharmaceutically active substances for use in mixtures have been encapsulated either to mask bad taste or to control the release in the body.

Hitherto medicins have been coated with polymers or with polymers in combination with plasticizers to control drug release (microencapsulation). Applied on granulates of a drug it retards the rate of dissolution.

The main way to control drug dissolution from microcapsules is the amount of polymer applied, in order to obtain the expected plasma profile of the drug. This can also be obtained by adding water soluble substances to the coat during the coating process.

Disclosure of the invention

The present invention provides a mixture, wherein bad taste of the drug is masked and/or it provides a mixture with retarded dissolution to obtain slow release effect.

The mixture is obtained either by suspending the dry powder in an aqueous solution or by suspending the microcapsules in a solution of the release controlling substance.

The drug release from the microcapsules within the mixture, here called leakage, is very low, but in the body the drug is released from the microcapsules and available for absorption.

This invention also provides for increased drug stability in the mixture.

This result is obtained by adding to the microencapsulated active substance and customary adjuvants a release-controlling substance (sink).

As sink can be used a carbohydrate or a carbohydrate-related compound, for instance a poly- or an oligosaccharide such as dextrane; a disaccharide such as saccharose, maltose or lactose; a monosaccharide such as glucose, fructose, galactose, mannose or xylitol; a carbohydrate-related compound such as mannitol, sorbitol, glycerol, glycol, a glycoside of a monosaccharide or a substance derived from ethyleneglycol for instance polyethyleneglycol (trade names Carbowaxes® and Carbopoles®).

As sink one or a mixture of two or more of the mentioned substances can be used.

The amount of sink should be between 60% and 99% (weight/weight), preferably 60—75% (weight/weight) of the entire preparation, that is of the ready to use suspension for oral administration (the mixture).

Sugars that can be used according to the invention are among others sucrose, glucose, fructose and sorbitol.

As pharmaceutically active substance any drug can be used, for instance any one of the following:

Chemotherapeutics: bacampicillin, ampicillin, flucloxacillin, tetracycline, dicloxacillin, chloramphenicol, gentamicin, erythromycin, lincomycin, rifampicin, sulphadiazine, sulphamethoxypyradazine, griseofulvine, nitrofurantoine.

Adrenergis and beta-receptor-stimulators: ephedrine, terbutaline, theophylline, enprophylline.

Expectorants and cough depressants: Ethylmorphine, dextromethorphan, noscapine, bromhexine.

Heartglucosides and antiarythymics: Digitoxine, digoxin, dispyramide, procainide, tocainide, alprenolol, atenolol, metoprolol, pindolol, propranolol.

Blood pressure depressants: betanidine, clonidine, guanetidine, methyldopa, reserpine trimetaphane, hydrolazine, bendropphlumetiazide, furosemide, chlorotiazide.

Antihistamines: brompheniramine, chlorcyclizine, chlorpheniramine, diphenhydramine, prometazine.

Peroral antidiabetes: carbutamide, chlorpropamide, tolazamide, tolbutamide.

Sedatives, hypnotics, antidepressants: hexobarbital, pentobarbital, phenobarbital, meprobamate, chlordiazepoxide, diazepam, flunitrazepam, nitrazepam, oxazepam, chlormethiazol, chlorpromazine, fluphenazine, perphenazine, prochlorperazin, haloperidol, lithium, alaproclate, zimeldine, amitryptiline, imipramine, nortripthyline.

Antiepileptics: phenytoine, ethotoin, ethosuximide, carbamazepine.

Analgetics, anesthetics: codeine, morphine, pentazocine, petidine, dextropropoxyphene, methadone, acetylsalicylic acid, diflunisal, phenazone, phenylbutazon, acetaminophene, indometazine, naproxen, piroxicam, lidocaine, etidocaine.

Others: cimetidine, quinidine, dicoumarine, warfarine, potassium chloride, chloroquine.

The preferred drug is bacampicillin hydrochloride (1'-ethoxycarbonyloxyethyl 6-[D(—)-2-amino-2-phenylacetamidol]-penicillanate hydrochloride), other epimeric forms and the racemic form of bacampicillin hydrochloride.

Other preferred drugs are theophylline, enprophylline and erythromycine.

The drugs mentioned above are used in neutral or salt form.

The following salts of the drugs mentioned above can be used:

Acetate, benzenesulfone, benzoate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, clycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulphate, tannate, tartrate, teoclate, triethiodide.

Also the further cationic salts can be used. Suitable cationic salts include the alkali metal, e.g. sodium and potassium, and ammonium salts and salts of amines known in the art to be pharmaceutically acceptable, e.g. glycine, ethylene diamine, choline, diethanolamine, triethanolamine, octadecylamine, diethylamine, triethylamine, 1-amino-2-propanol-2-amino-2-(hydroxymethyl)propane-1,3-diol and 1-(3,4-dihydroxyphenyl)-2-isopropylaminoethanol.

Coating material

Polymers:

Synthetic polymers of polyvinyl type, e.g. polyvinylchloride, polyvinylacetate, polyvinylalcohol.

Polyethylene type, e.g. polyethylene, polystyrene.

Polymers of acrylic acid or acrylic acid ester type, e.g. methylmetacrylate or copolymers of acrylic monomers.

Biopolymers or modified biopolymers of cellulose, e.g. ethylcellulose, cellulose acetate phtalate.

The polymer can be water insoluble, acid soluble or alkaline soluble and mixed with plasticizer or other filler and water soluble modified biopolymer, e.g. hydroxy-propyl-cellulose.

Also fats and oils, wax, higher fatty acids, higher alcohols or polyhydric alcohols can be used as such or in combination.

In one embodiment of the invention bacampicillin hydrochloride (BAPC) is encapsulated in an insoluble, microporous polymer, such as ethyl cellulose and sucrose is used as sink to make a dry powder for mixture, which is then dissolved in water to make a mixture.

In another embodiment of the invention BAPC is encapsulated in a polymer soluble in acid, such as Eudragit® E 100 and sucrose is used as sink to make a dry powder for mixture, which is then dissolved in water to make a mixture.

In a further embodiment of the invention theophylline is microencapsulated in a shell of ethyl cellulose and sorbitol is used to sink to make a dry powder for mixture, which is then dissolved in water to make a mixture.

In a further embodiment of the invention acetylsalcylic acid is encapsulated in a shell of cellulose acetate phtalate and sucrose is used as sink to make a dry powder for mixture, which is then dissolved in water to make a mixture.

A release controlling substance is mixed with other constituents and microcapsules of the drug are added to this dry powder and mixed in a conventional blender. This dry powder is then added to bottles in a filling machine. Water is then added, by the customer or at the pharmacy, to dissolve the release controlling substance.

Alternatively, a solution of the release controlling substance and other consittuents is prepared. The microcapsules of the drug can then be added either to this solution and then filled into bottles ready to use, or the microcapsules of the drug can be filled into a separate container and be added by the customer or the pharmacy to the solution prior to use.

Best mode of carrying out the invention

Leakage studies

Leakage studies were carried out in order to show that the microcapsules will not release any significant amount of the drug into the sink causing bad taste in contact with water, causing degradation or losing its ability to work as controlled release formulation.

## EP 0 101 418 B1

Microcapsules were added to sink solution according to the invention. The amount of drug which had been released from the microcapsules was analyzed. This is called leakage. The samples were in some instances stored up to 80 days at room temperature. The sink was analyzed spectrophotometrically. The result is given in percent leakage which is the amount of the drug which is in solution divided by the initial amount of microencapsulated drug.

In order to demonstrate the effect of the release controlling substance the release studies were also performed in water. Microcapsules were placed in a beaker and water was added. The stirring rate was 30 rpm and the amount of release was calculated as described above.

Example 1

25.8 g of pharmaceutical mixture contains

| | |
|---|---|
| Bacampicillin hydrochloride | 0.80 g |
| Eudragit® E 100 microcapsules (64% drug) | |
| Fructose | 18.75 g |
| Water | 6.25 g |

Fructose was dissolved in water before the microcapsules were added.
The mixture contains 67.5% release controlling substances.

| Time (hours) | Leakage (%) |
|---|---|
| 2 | <0.2 |

| Time (hours) | Release in water (%) |
|---|---|
| 0.008 | 50 |
| 0.05 | 90 |

Example 2

31.3 g of pharmaceutical mixture contains:

| | |
|---|---|
| Theophylline ethyl cellulose microcapsules (72% drug) | 0.05 g |
| a { Fructose | 23.44 g |
| { Water | 7.82 g |
| b { Sorbitol | 20.94 g |
| { Water | 7.82 g |

The two mixtures were prepared according to Example 1.
The mixture contain a) 75% b) 72% release controlling substance.

| Time (days) | Leakage (%) a) | b) |
|---|---|---|
| 1 | <0.2 | 0.7 |
| 3 | <0.2 | — |
| 5 | <0.2 | — |
| 7 | <0.2 | — |
| 10 | <0.2 | — |

| Time (days) | Release in water (%) |
|---|---|
| 0.21 | 50 |
| 0.33 | 90 |

4

Example 3

31.3 g of pharmaceutical mixture contains:

| | | |
|---|---|---|
| Theophylline ethyl cellulose microcapsules (72% drug) | | 0.05 g |
| a { | Sucrose | 9.38 g |
| | Sorbitol | 9.38 g |
| b { | Sucrose | 9.38 g |
| | Glycerol | 9.38 g |
| c { | Glucose | 9.38 g |
| | Fructose | 9.38 g |
| | Water | 12.5 g |

The three mixtures were prepared according to Example 1.

The mixtures contain 60% of release controlling substances.

| Time (days) | Leakage (%) | | |
|---|---|---|---|
| | a) | b) | c) |
| 1 | 0.20 | <0.2 | 0.26 |
| 2 | 0.31 | 0.35 | 0.28 |
| 5 | 0.65 | 0.82 | 0.49 |
| 9 | 1.15 | 1.77 | 0.90 |

| Time (days) | Release in water (%) |
|---|---|
| 0.21 | 50 |
| 0.33 | 90 |

Example 4

75.1 g of pharmaceutical mixture contains:

| | |
|---|---|
| Acetylic salicylic acid cellulose acetate phtalate microcapsules (69% drug) | 0.100 g |
| Sucrose | 48.75 g |
| Phosphate buffer (pH 7.0) | 26.25 g |

Sucrose was dissolved in the phosphate buffer. The microcapsules were then added.

The mixture contains 65% release controlling substance.

| Time (days) | Leakage (%) |
|---|---|
| 1 | 3.5 |

| Time (days) | Release in phosphate buffer pH 7.0 (%) |
|---|---|
| 0.008 | 50 |
| 0.017 | 90 |

Example 5

| | a | b |
|---|---|---|
| Becampicillin hydrochloride ethyl cellulose microcapsules (70% drug) | 0.27 g | 0.27 g |
| Sodium bicarbonate | 0.40 g | — |
| Water | 5.0 g | 5.0 g |

| Time (days) | Leakage (%) | |
|---|---|---|
| | a | b |
| 1 | 85 | 100 |

Example 6

Four different microcapsules coated with ethylcellulose were suspended in sorbitol dissolved in water according to following composition.

| Microcapsules | 50 mg |
|---|---|
| Sorbitol | 45.1 g |
| Water | 19.3 g |

The mixture contain 70% release controlling substance.

| Microcapsules | | Leakage in sorbitol sink | | Release in water | |
|---|---|---|---|---|---|
| | | (%) | (days) | (%) | (h) |
| KCl | (86)* | 16 | 21 | 56 | 3 |
| Paracetaminophene | (91)* | 19 | 21 | 35 | 1 |
| Flucloxacillin | (89)* | 20 | 1 | 90 | 0.5 |
| Fenoxymethyl penicillin potassium | (83)* | 10 | 1 | 80 | 1 |

*content of active drug in the microcapsules

Example 7

0.2 g theophyllin microcapsules according to Example 2 were suspended in different sugar solutions.

| Release controlling substance % (w/w) | | Leakage (%) | Time (days) |
|---|---|---|---|
| Sorbitol | 70 | 3 | 80 |
| Fructose | 75 | 3 | 80 |
| Fructose-xylitol | 19—41 | 10 | 80 |
| Fructose-xylitol | 38—28 | 6 | 80 |
| Fructose-xylitol | 56—14 | 4 | 80 |

It is thus possible to restrict the leakage in the mixture to only a few percent after almost three months storage in room temperature.

Example 8

65.4 g of pharmaceutical mixture contains:

| Theophyllin wax coated microcapsules (52% drug) | 1 g |
|---|---|
| Sorbitol | 45.1 g |
| Water | 19.3 g |

The mixture was prepared according to Example 2.

The mixture contains 69% release controlling substance.

| Time (days) | Leakage (%) |
| --- | --- |
| 22 | 0.7 |

| Time (days) | Release in water (%) |
| --- | --- |
| 0.5 | 19 |

Example 9

26.31 g of pharmaceutical mixture contains

| | |
| --- | --- |
| Prochloroperazin wax coated microcapsules (3.4% drug) | 10 mg |
| Sorbitol | 18 g |
| Water | 8.3 |

The mixture was prepared according to Example 2
The mixture contains 70% release controlling substance.

| Time (days) | Leakage (%) |
| --- | --- |
| 12 | 2.7 |

| Time (days) | Release in water (%) |
| --- | --- |
| 0.25 | 28 |

Example 10

27.15 g of pharmaceutical mixture contains:

| | |
| --- | --- |
| Theophylline ethyl cellulose coated microcapsules (72%) | 0.15 g |
| Polyethyleneglycol (Carbowax® 400) | 20.25 g |
| Water | 6.75 g |

Polyethyleneglycol was mixed with water and the microcapsules were added.
The mixture contains 75% release controlling substance.

| Time (days) | Leakage (%) |
| --- | --- |
| 15 | 2.4 |

The release in water, see Example 2.

Example 11

13.877 g of pharmaceutical mixture contains:

| | |
| --- | --- |
| Erythromycin cellulose acetate phtalate coated microcapsules (57% drug) | 0.877 mg |
| Fructose | 9.75 |
| Water | 3.25 |

The microcapsules were added to a solution of fructose in water.

# EP 0 101 418 B1

The mixture contains 71% release controlling substance.

| Time (days) | Leakage (%) |
|---|---|
| 10 | <1 |

| Time (days) | Release in water (%) |
|---|---|
| 0.25 | 46 |

Release studies

Microcapsules were suspended in 75% release controlling substance solution and after two or three days storage the microcapsules were filtered off and the release of the drug was measured. The microcapsules were placed in a beaker containing either simulated gastric fluid or simulated intestinal fluid at 37°C in order to simulate the in vivo situation. The stirring rate was 30 rpm. Samples were withdrawn after certain time points and those were analyzed for drug content spectrophotometrically.

The results show time to obtain 50, 70 and 90 percent release of the total amount of microencapsulated drug.

### Theophylline microcapsules

| Release (%) | Simulated gastric fluid (hours) | | Stimulated intestinal fluid (hours) | |
|---|---|---|---|---|
| | Original | Stored 3 days | Original | Stored 2 days |
| 50 | 4.2 | 4.4 | 3.7 | 4.4 |
| 70 | 5.7 | 5.8 | 5.5 | 6.6 |
| 90 | 6.2 | 6.4 | 7.5 | 8.3 |

### Acetylic salicylic acid

| Release (%) | Simulated gastric fluid (%) | | Stimulated intestinal fluid (hours) | |
|---|---|---|---|---|
| | Original | Stored 2 days | Original | Stored 2 days |
| 50 | | | 0.14 | 0.21 |
| 70 | | | 0.22 | 0.31 |
| 90 | | | 0.3 | 0.5 |
| 1 h | 12% | 8% | | |
| 2 h | 25% | 15% | | |

### Bacampicillin hydrochloride Eudragit® E 100 microcapsules

| Release (%) | Simulated gastric fluid (min) | | Simulated intestinal fluid (min) | |
|---|---|---|---|---|
| | Original | Stored 2 days | Original | Stored 2 days |
| 50 | 0.4 | 0.8 | 1.5 | 3 |
| 70 | 0.5 | 0.9 | 1.8 | 3.7 |
| 90 | 0.7 | 1.0 | 2.5 | 5 |

Microcapsule compositions as in Examples 6, 8 and 10.

8

| Microcapsules | Release in water | | | | |
|---|---|---|---|---|---|
| | Initially | | Storage time | | |
| | (%) | (h) | (days) | (%) | (h) |
| KCl | 56 | 3 | 14 | 53 | 3 |
| Paracetaminophene ⎤ | 35 | 1 | 14 | 48 | 1 |
| Fenoxymethyl ⎬ a) | 80 | 1 | 3 | 81 | 1 |
| penicillin potassium ⎦ | | | | | |
| Theophyllin wax coated b) | 19 | 12 | 25 | 17 | 12 |
| Theophyllin ethyl cellulose coated c) | 46 | 6 | 6 | 50 | 6 |

a) according to Example 6
b) according to Example 8
c) according to Example 10

Release studies have also been carried out on the compositions in Example 7. The release rate was performed according to USP XX (method II paddle) 100 rpm in 900 ml 37° water.

The release rate is expressed as percent released per hour. The initial release rate was 12%/h.

| Release controlling substance | Release rate (%/h) | Time (days) |
|---|---|---|
| Sorbitol | 11.7 | 80 |
| Fructose | 11.8 | 80 |
| Fructose-xylitol (19—41) | 10.5 | 80 |
| Fructose-xylitol (38—28) | 11.9 | 80 |
| Fructose-xylitol (56—14) | 11.9 | 80 |

The influence on storage time of the microcapsules in the different sink solution is negligible.

Stability studies

Microcapsule suspensions were prepared with sink solutions according to the invention. The suspensions were stored and the drug content was measured with HPLC analysis as a selective and precise method.

Mixtures not according to the invention ⎰ Mixtures
a) According to Example 6a
b) According to Example 6b

c) Bacampicillin HCl microcaps. (72% drug)      0.36
   ethylcellulose coated
   Sucrose      8.32
   Water      4.48

d) Bacampicillin HCl microcaps. (72% drug)      0.36
   ethyl cellulose coated
   Fructose      9.6
   Water      3.2

e) Acetyl salicylic acid microcaps. (69% drug)      0.72
   cellulose acetate phtalate coated
   Sucrose      8.32
   Citrate buffer pH 3      4.48

f) Erythromycin microcaps. (87% drug)      0.44 g
   cellulose acetate phtalate coated
   Phosphate buffer pH 7.0      4.48 g
   Sucrose      8.32 g

| | Storage condition | | |
| Mixture | Time (days) | Temp. (°C) | Intact drug* (%) |
|---|---|---|---|
| a | 1 | 25 | 2 |
| b | 1 | 25 | 60 |
| c | 7 | 25 | 83 |
| d | 7 | 25 | 89 |
| e | 30 | 50 | 70 |
| f | 30 | 50 | 82 |

*initially the amount of intact drug was 100%.

The results imply that mixtures according to the invention has an improving effect on the stability of drugs.

**Claims for the Contracting States: BE, CH, DE, FR, IT, LI, NL and SE**

1. A ready-to-use pharmaceutical suspension with controlled release of active substance including masking of bad taste and increasing the stability of active substance characterized in that it contains a microencapsulated active substance combined outside the encapsulation with 60—99% (weight/weight) of the ready-to-use suspension, preferably 60—75%, of a release controlling substance which is a carbohydrate selected from polysaccharides, oligosaccharides, disaccharides and monosaccharides or a carbohydrate-related compound selected from mannitol, sorbitol, glycerol, glycol, a glycoside of a monosaccharide and a substance derived from ethyleneglycol or a mixture of such compounds.

2. A ready-to-use pharmaceutical suspension according to claim 1, characterized in that the release controlling substance is sucrose, glucose, fructose or sorbitol.

3. A ready-to-use pharmaceutical suspension according to claim 1, characterized in that the active substance is bacampicillin or theophylline.

4. A method for preparing a ready-to-use pharmaceutical suspension with controlled release of active substance, which comprises

a) mixing a microencapsulated active substance with customary adjuvants and a further substance for control of the release of active substance which further substance is a carbohydrate selected from polysaccharides, oligosaccharides, disaccharides and monosaccharides or a carbohydrate-related compound selected from mannitol, sorbitol, glycerol, glycol, a glycoside of a monosaccharide and a substance derived from ethyleneglycol or a mixture of such compounds in an amount of 60—99% (weight/weight), preferably 60—75% (weight/weight) of the ready-to-use suspension, and thereafter adding water to obtain the ready-to-use suspension or

b) mixing a microencapsulated active substance with a solution of customary adjuvants and a further substance which further substance is a carbohydrate selected from polysaccharides, oligosaccharides, disaccharides and monosaccharides or a carbohydrate-related compound selected from mannitol, sorbitol, glycerol, glycol, a glycoside of a monosaccharide and a substance derived from ethyleneglycol or a mixture of such compounds in an amount of 60—99% (weight/weight), preferably 60—75% (weight/weight) of the ready-to-use suspension.

**Claims for the Contracting State: AT**

1. A method for preparing a ready-to-use pharmaceutical suspension with controlled release of active substance including masking of bad taste and increasing the stability of active substance, which comprises

a) mixing a microencapsulated active substance with customary adjuvants and a further substance for control of the release of active substance which further substance is a carbohydrate selected from polysaccharides, oligosaccharides, disaccharides and monosaccharides or a carbohydrate-related compound selected from mannitol, sorbitol, glycerol, glycol, a glycoside of a monosaccharide and a substance derived from ethyleneglycol or a mixture of such compounds in an amount of 60—99% (weight/weight), preferably 60—75% (weight/weight) of the ready-to-use suspension, and thereafter adding water to obtain the ready-to-use suspension or

b) mixing a microencapsulated active substance with a solution of customary adjuvants and a further substance which further substance is a carbohydrate selected from polysaccharides, oligosaccharides, disaccharides and monosaccharides or a carbohydrate-related compound selected from mannitol, sorbitol, glycerol, glycol, a glycoside of a monosaccharide and a substance derived from ethyleneglycol or a mixture of such compounds in an amount of 60—99% (weight/weight), preferably 60—75% (weight/weight) of the ready-to-use suspension.

2. A method according to claim 1, characterized in that the release controlling substance is sucrose, glucose, fructose or sorbitol.

3. A method according to claim 1, characterized in that the active substance is bacampicillin or theophylline.

**Patentansprüche für die Vertragsstaaten: BE, CH, DE, FR, IT, LI, LU, NL, SE**

1. Verwendungsbereite pharmazeutische Suspension mit gesteuerter Abgabe einer aktiven Substanz, einschließlich der Maskierung des üblen Geschmacks und der Erhöhung der Stabilität der aktiven Substanz, dadurch gekennzeichnet, daß sie eine mikroverkapselte aktive Substanz enthält, die außerhalb der Verkapselung mit 60—99% (Gew./Gew.) der verwendungsbereiten Suspension, vorzugsweise 60—75%, einer die Abgabe steuernden Substanz kombiniert ist, die ein Kohlehydrat, ausgewählt aus Polysacchariden, Oligosacchariden, Disacchariden und Monosacchariden, oder eine Kohlehydrat-verwandte Verbindung, ausgewählt aus Mannit, Sorbit, Glyzerin, Glykol, einem Glykosid eines Monosaccharids und einer von Äthylenglykol abgeleiteten Substanz, oder eine Mischung solcher Verbindungen ist.

2. Verwendungsbereite pharmazeutische Suspension nach Anspruch 1, dadurch gekennzeichnet, daß die die Abgabe steuernde Substanz Saccharose, Glukose, Fruktose oder Sorbit ist.

3. Verwendungbereite pharmazeutische Suspension nach Anspruch 1, dadurch gekennzeichnet, daß die aktive Substanz Bacampicillin oder Theophyllin ist.

4. Verfahren zur Herstellung einer verwendungsbereiten pharmazeutischen Suspension mit gesteuerter Abgabe einer aktiven Substanz, welches umfaßt:

a) das Mischen einer mikroverkapselten aktiven Substanz mit üblichen Adjuvantien und einer weiteren Substanz zur Steuerung der Abgabe der aktiven Substanz, welche weitere Substanz ein Kohlehydrat, ausgewählt aus Polysacchariden, Oligosacchariden, Disacchariden und Monosacchariden, oder eine Kohlehydrat-verwandte Verbindung, ausgewählt aus Mannit, Sorbit, Glyzerin, Glykol, einem Glykosid eines Monosaccharids und einer von Äthylenglykol abgeleiteten Substanz, oder eine Mischung solcher Verbindungen ist, in einer Menge von 60—99% (Gew./Gew.), vorzugsweise 60—75% (Gew./Gew.), der verwendungsbereiten Suspension, und die nachfolgende Zugabe von Wasser zum Erhalt der verwendungsbereiten Suspension, oder

b) das Mischen einer mikroverkapselten aktiven Substanz mit einer Lösung herkömmlicher Adjuvantien und einer weiteren Substanz, welche weitere Substanz ein Kohlehydrat, ausgewählt aus Polysacchariden, Oligosacchariden, Disacchariden und Monosacchariden, oder eine Kohlehydratverwandte Verbindung, ausgewählt aus Mannit, Sorbit, Glyzerin, Glykol, einem Glykosid eines Monosaccharids und einer von Äthylenglykol abgeleiteten Substanz, oder eine Mischung solcher Verbindungen ist, in einer Menge von 60—99% (Gew./Gew.), vorzugsweise 60—75% (Gew./Gew.), der verwendungsbereiten Suspension.

**Patentansprüche für den Vertragsstaat: AT**

1. Verfahren zur Herstellung einer verwendungsbereiten pharmazeutischen Suspension mit gesteuerter Abgabe einer aktiven Substanz, einschließlich der Maskierung des üblen Geschmacks und der Erhöhung der Stabilität der aktiven Substanz, welches umfaßt:

a) das Mischen einer mikroverkapselten aktiven Substanz mit üblichen Adjuvantien und einer weiteren Substanz zur Steuerung der Abgabe der aktiven Substanz, welche weitere Substanz ein Kohlehydrat, ausgewählt aus Polysacchariden, Oligosacchariden, Disacchariden und Monosacchariden, oder eine Kohlehydrat-verwandte Verbindung, ausgewählt aus Mannit, Sorbit, Glyzerin, Glykol, einem Glycosid eines Monosaccharids und einer von Äthylenglykol abgeleiteten Substanz, oder eine Mischung solcher Verbindungen ist, in einer Menge von 60—99% (Gew./Gew.), vorzugsweise 60—75% (Gew./Gew.), der verwendungsbereiten Suspension, und die nachfolgende Zugabe von Wasser zum Erhalt der verwendungsbereiten Suspension, oder

b) das Mischen einer mikroverkapselten aktiven Substanz mit einer Lösung herkömmlicher Adjuvantien und einer weiteren Substanz, welche weitere Substanz ein Kohlehydrat, ausgewählt aus Polysacchariden, Oligosacchariden, Disacchariden und Monosacchariden, oder eine Kohlehydrat-verwandte Verbindung, ausgewählt aus Mannit, Sorbit, Glyzerin, Glykol, eine Glykosid eines Monosaccharids und einer von Äthylenglykol abgeleiteten Substanz, oder eine Mischung solcher Verbindungen ist, in einer Menge von 60—99% (Gew./Gew.), vorzugsweise 60—75% (Gew./Gew.), der verwendungsbereiten Suspension.

2. Verfahren nach Anspruch 1, dadurch gekennzeichnet, daß die die Abgabe steuernde Substanz Saccharose, Glukose, Fruktose oder Sorbit ist.

3. Verfahren nach Anspruch 1, dadurch gekennzeichnet, daß die aktive Substanz Bacampicillin oder Theophyllin ist.

# EP 0 101 418 B1

**Revendications pour les Etats Contractants: BE, CH, DE, FR, IT, LI, LU, NL, SE**

1. Suspension pharmaceutique prête à l'emploi avec libération contrôlée de substance active, comprenant le masquage de mauvais goût et l'augmentation de la stabilité de la substance active, caractérisée en ce qu'elle contient une substance active microencapsulée, combinée, en dehors de l'encapsulation, avec 60—99% (poids/poids) de la suspension prête à l'emploi, de préférence de 60—75%, d'une substance contrôlant la libération qui est un hydrate de carbone choisi parmi les polysaccharides, les oligosaccharides, les disaccharides et les monosaccharides, ou un composé apparenté à un hydrate de carbone choisi parmi le mannitol, le sorbitol, le glycérol, le glycol, un glycoside d'un monosaccharide et une substance dérivée de l'éthylèneglycol ou un mélange de ces composés.

2. Suspension pharmaceutique prête à l'emploi selon la revendication 1, caractérisée en ce que la substance contrôlant la libération est le saccharose, le glucose, le fructose ou le sorbitol.

3. Suspension pharmaceutique prête à l'emploi selon la revendication 1, caractérisée en ce que la substance active est la bacampicilline ou la théophylline.

4. Procédé de préparation d'une suspension pharmaceutique prête à l'emploi avec libération contrôlée de substance active, qui comprend

a) le mélange d'une substance active microencapsulée avec des adjuvants courants et une autre substance pour contrôler la libération de la substance active, cette autre substance étant un hydrate de carbone choisi parmi les polysaccharides, les oligosaccharides, les disaccharides et les monosaccharides, ou un composé apparenté à un hydrate de carbone choisi parmi le mannitol, le sorbitol, le glycérol, le glycol, un glycoside d'un monosaccharide et une substance dérivée de l'éthylèneglycol ou un mélange de ces composés, en une quantité de 60—99% (poids/poids), de préférence de 60—75% (poids/poids) de la suspension prête à l'emploi, et ensuite l'addition d'eau pour obtenir une suspension prête à l'emploi, ou

b) le mélange d'une substance active microencapsulée avec une solution d'adjuvants courants et d'une autre substance, cette autre substance étant un hydrate de carbonate choisi parmi les polysaccharides, les oligosaccharides, les disaccharides et les monosaccharides, ou un composé apparenté à un hydrate de carbonate choisi parmi le mannitol, le sorbitol, le glycérol, le glycol, un glycoside d'un monosaccharide et une substance dérivée de l'éthylèneglycol ou un mélange de ces composés, en une quantité de 60—99% (poids/poids), de préférence de 60—75% (poids/poids) de la suspension prête à l'emploi.

**Revendications pour l'Etat Contractant: AT**

1. Procédé de préparation d'une suspension pharmaceutique prête à l'emploi avec libération contrôlée de substance active, comprenant le masquage du mauvais goût et l'augmentation de la stabilité de la substance active, qui comprend

a) le mélange d'une substance active microencapsulée avec des adjuvants courants et une autre substance pour contrôler la libération de la substance active, cette autre substance étant en hydrate de carbone choisi parmi les polysaccharides, les oligosaccharides, les disaccharides et les monosaccharides, ou un composé apparenté à un hydrate de carbone choisi parmi le mannitol, le sorbitol, le glycérol, le glycol, un glycoside d'un monosaccharide et une substance dérivée de l'éthylèneglycol ou un mélange de ces composés, en une quantité de 60—99% (poids/poids), de préférence de 60—75% (poids/poids) de la suspension prête à l'emploi, et ensuite l'addition d'eau pour obtenir une suspension prête à l'emploi, ou

b) le mélange d'une substance active microencapsulée avec une solution d'adjuvants courants et d'une autre substance, cette autre substance étant un hydrate de carbone choisi parmi les polysaccharides, les oligosaccharides, les disaccharides et les monosaccharides, ou un composé apparenté à un hydrate de carbone choisi parmi le mannitol, le sorbitol, le glycérol, le glycol, un glycoside d'un monosaccharide et une substance dérivée de l'éthylèneglycol ou un mélange de ces composés, en une quantité de 60—99% (poids/poids), de préférence de 60—75% (poids/poids) de la suspension prête à l'emploi.

2. Procédé selon la revendication 1, caractérisé en ce que la substance contrôlant la libération est le saccharose, le glucose, le fructose ou le sorbitol.

3. Procédé selon la revendication 1, caractérisé en ce que la substance active est la bacampicilline ou la théophylline.